(19) Europäisches Patentamt

European Patent Office

Office européen des brevets

(11) **EP 1 205 190 B1**

(12) **EUROPEAN PATENT SPECIFICATION**

(45) Date of publication and mention
of the grant of the patent:
**03.05.2006 Bulletin 2006/18**

(51) Int Cl.:
*A61K 47/02* (2006.01)     *A61K 47/32* (2006.01)
*A61K 9/22* (2006.01)     *A61K 9/32* (2006.01)

(21) Application number: **00948291.0**

(22) Date of filing: **01.08.2000**

(86) International application number:
**PCT/JP2000/005075**

(87) International publication number:
**WO 2001/010466 (15.02.2001 Gazette 2001/07)**

(54) **STABLE MEDICINAL COMPOSITIONS FOR ORAL USE USING FERRIC OXIDES**

STABILE MEDIZINISCHE ZUSAMMENSETZUNGEN ZUR ORALEN VERABREICHUNG UNTER VERWENDUNG VON EISENOXIDEN

COMPOSITIONS MEDICINALES STABLES POUR ADMINISTRATION ORALE EN UTILISANT DES OXYDES DE FER

(84) Designated Contracting States:
**AT BE CH CY DE DK ES FI FR GB GR IE IT LI LU MC NL PT SE**

(30) Priority: **04.08.1999 US 147222 P**

(43) Date of publication of application:
**15.05.2002 Bulletin 2002/20**

(73) Proprietor: **Astellas Pharma Inc.
Chuo-ku,
Tokyo (JP)**

(72) Inventors:
• **SAKO, Kazuhiro**
**Yaizu-shi, Shizuoka 425-0072 (JP)**
• **SAWADA, Toyohiro**
**Yaizu-shi, Shizuoka 425-0072 (JP)**
• **YOSHIHARA, Keiichi**
**Yaizu-shi, Shizuoka 425-0072 (JP)**
• **YOSHIOKA, Tatsunobu**
**Yaizu-shi, Shizuoka 425-0072 (JP)**
• **WATANABE, Shunsuke**
**Yaizu-shi, Shizuoka 425-0072 (JP)**

(74) Representative: **Geering, Keith Edwin et al
REDDIE & GROSE
16 Theobalds Road
London WC1X 8PL (GB)**

(56) References cited:
**EP-A1- 0 901 787       WO-A-96/32097
WO-A-99/06045       DE-A- 3 520 184
JP-A- 4 346 929       JP-A- 5 092 918
US-A- 4 859 470       US-A- 5 322 698**

Remarks:
The file contains technical information submitted after the application was filed and not included in this specification

**Description**

TECHNICAL FEILD

The present invention relates to a stable solid pharmaceutical composition for oral use that prevents changes in drug release in a matrix type sustained-release preparation containing polyethylene oxide. The present invention also relates to a method of producing a stable solid pharmaceutical composition for oral use that prevents changes in drug release in a matrix type sustained-release preparation containing polyethylene oxide. Furthermore, the present invention relates to a method of preventing changes in drug release in a matrix type sustained-release preparation containing polyethylene oxide.

BACKGROUND OF THE INVENTION

Various sustained-release preparations have been developed in the field of pharmaceuticals, and release of the drug from this preparation is an important factor of preparation design in terms of *in vivo* absorption of the drug when the preparation is orally taken by the patient. Stable drug release from the preparation, wherein the release profile is steady with few changes, is essential, from the time the pharmaceutical preparation is made until it is transported, stored and orally taken by the patient.

Incidentally, various sustained-release preparations have been made by researchers affiliated with the applicant of the present application. It goes without saying that, similar to the main agent, in sustained-release preparations (WO 94/06414), the polymer substance that forms a hydrogel as the main base in particular must also be stable in order to maintain the drug release rate.

Polyethylene oxide can be mentioned as one polymer substance that forms a hydrogel. This substance is a water-soluble thermoplastic resin in the form of white powder or granules that is obtained by polymerization of ethylene oxide and that has a molecular weight of from several 100,000s to several 1,000,000s. It is known that because it is extremely sticky when wet, sustained-release preparations containing this substance show good drug release profile in the digestive tract. Although it is a known fact that the erosion rate of the polymer substance or a matrix made from said substance has a strong effect on this drug release rate, the factors that affect stability of the polyethylene oxide used as the base for sustained-release preparations, particularly factors that affect drug release profile, are not known.

The patent pubticabons WO9632097 ,DE3520184, US5322698 ,WO9906045, Jap4346929. JP5092918, EP0901787, cited in the international and european search reports, do not describe a combination of a hydrophilic base dispersed in a high molecular weight polyethylene oxide. Furthermore ferric oxide oxide, when used or suggested, is used as a colorant or an agent for protecting a light-sensitive drug per se from degradation, but not for protecting a sustained-release matrix against storage-induced acceleration of drug release as described in present claim 12. US4859470 describes an osmotic device comprising a drug layer and a push composition containing polyethylene oxide with a MW of 7,000,000, a hydrophilic base such as NaCl and HPMC and ferric oxide. However the drug is not dispersed in the push composition.

The present inventors performed studies on polyethylene oxide as the base for sustained-release preparations, and they discovered that erosion of the matrix made from polyethylene oxide is accelerated when the preparation is stored under exposure to light. As a result, the drug release rate is accelerated with time, and there are changes in drug release profile, and its prevention has been desired.

DISCLOSURE OF THE INVENTION

Objects of the present invention are to provide a stable preparation that shows no changes in drug release profile in matrix type sustained-release preparations containing polyethylene oxide during storage under exposure to light, provide a method of producing a stable preparation with which there are no changes in drug release profile in matrix type sustained-release preparations containing polyethylene oxide; and to prevent changes in drug release profile in matrix type sustained-release preparations containing drugs and polyethylene oxide during storage under exposure to light.

Under the above circumstances, the present inventors made extensive studies and discovered that changes in drug release profile can be prevented during storage even under exposure to light, if yellow ferric oxide or red ferric oxide (pharmaceutical additives previously used as coloring agent in an amount of less than 0.1 wt.%) is physically mixed in an amount of 10 wt%, (which is in excess of the very small amount used as coloring agent) with a drug and polyethylene oxide in a matrix type sustained release tablet. The present inventors made further studies and completed the present invention upon discovering that changes in drug release profile of a preparation can be prevented by adding yellow ferric oxide or red ferric oxide not only by means of physical mixing, but also by means of coating a tablet. The mechanism of preventing effect by adding yellow ferric oxide or red ferric oxide has not been elucidated, but it is considered that the mechanism is not a mere prevention of the degradation of polyethylene oxide by exposure to light. That is, the present inventors found that titanium oxide absorbing light at the wavelength of not more than 400 nm (UV) and reflecting light at the wavelength of not less than 400 nm, or medicinal carbon absorbing light at all wavelength (broad range) cannot reduce changes in drug release in a matrix type tablet consisting of polyethylene oxide and polyethylene glycol even if it was added by physical mixing in an amount of 10 % per tablet weight. It was expected that the stability against light can be maintained if the wavelength (color) that influence on the stability of polyethylene oxide is blocked. Tb the contrary, changes in elution profile of the tablet could not be reduced even if all visible light was blocked by reflecting or by absorbing. This is because that it was considered that the special wavelength affecting the influence on the stability in the visible region does not exist because the color of the polyethylene oxide aqueous solution was colorless and clear and thus there is no special wavelength that influence the stability in the visible region. This is also because that, even if the special wavelength affecting the influence on the stability exists in the visible region, it was expected that selection of the additive reflecting all visible light (white color) or the additive absorbing all visible light (black color) can make the stable preparation with no changes in drug release profile. To the contrary, it was an unexpected result. Therefore, although there is/are unknown factor(s) affecting changes in drug release profile other than light, the results suggested that the addition of a specific amount of yellow ferric oxide, or red ferric oxide in a matrix type sustained-release preparation containing at least polyethylene oxide by compounding, by physical mixing or by coating tablet, are effective against the factors that change the drug release profile. Accordingly, the present invention relates to 1) a stable pharmaceutical composition for oral use which comprises a drug, a hydrophilic base, and polyethylene oxide and which is a matrix type sustained-release preparation, characterized by comprising a yellow ferric oxide and/or a red ferric oxide in an effective amount to stabilize the matrix type sustained-release preparation containing a drug, a hydrophilic base, and polyethylene oxide. Moreover, the present invention relates to 2) the stable pharmaceutical composition for oral use according to the above-mentioned 1), wherein the amount of a yellow ferric oxide and/or a red ferric oxide added is not less than 0.3 wt% per tablet weight. The present invention also relates to 3) the stable pharmaceutical composition for oral use according to the above-mentioned 1) or 2), wherein the amount of a yellow ferric oxide added is from 1 to 20 wt% per preparation weight. In addition, the present invention relates to 4) the stable pharmaceutical composition for oral use according to the above-mentioned 1) or 2), wherein the amount of red ferric oxide added is from 5 to 20 wt% per preparation weight. The present invention relates to 5) a method of producing a stable pharmaceutical composition for oral use comprising adding yellow ferric oxide and/or red ferric oxide in an amount effective to stabilize the matrix type sustained-release preparation to a drug, a hydrophilic base, and polyethylene oxide. Moreover, the present invention relates to 6) the method of producing a stable pharmaceutical composition for oral use according to the above-mentioned 5), wherein yellow ferric oxide and/or red ferric oxide is added to the preparation by at least one means selected from the group consisting of film coating, granulation and mixing. Furthermore, the present invention relates to 7) the method of producing a stable pharmaceutical composition for oral use according to the above-mentioned 5) or 6), wherein the amount of yellow ferric oxide and/or red ferric oxide added is not less than 0.3 wt% per tablet weight. The present invention also relates to 8) a method of preventing changes in drug release profile by adding yellow ferric oxide and/or red ferric oxide in an amount effective to stabilize a matrix type sustained-release preparation containing a drug, a hydrophilic base, and polyethylene oxide. The present invention further relates to 9) a use of yellow ferric oxide and/or red ferric oxide in an amount effective to stabilize a matrix type sustained-release preparation containing a drug, a hydrophilic base, and polyethylene oxide in order to prevent changes in drug release profile.

The term 'a matrix preparation' signifies that it is a preparation that contains polyethylene oxide as a base of a sustained-release preparation, wherein a drug and a hydrophilic base are dispersed in said polyethylene oxide.

There are no special restrictions to the drug used in the present invention as long as it is a drug used in sustained-release preparations that contain polyethylene oxide as one of its base components. Anti-inflammatory, antipyretic anti-spasmodics or analgesics such as indomethacin, diclofenac, diclofenac sodium, codeine, ibuprofen, phenylbutazone, oxyphenbutazone, mepirizole, aspirin, ethenzamide, acetaminophen, aminopyrine, phenacetin, butylscopolamine bromide, morphine, etomidoline, pentazocine, fenoprofen calcium, naproxen, celecxib, valdecoxib, and toramadol, anti-rheumatism drugs such as etodolac, anti-tuberculosis drugs such as isoniazid and ethambutol hydrochloride, cardiovascular drugs such as isosorbide dinitrate, nitroglycerin, nifedipine, barnidipine hydrochloride, nicardipine hydrochloride, dipyridamole, amrinone, indenolol hydrochloride, hydralazine hydrochloride, methyldopa, furosemide, spironolactone, guanethidine nitrate, reserpine, amosulalol hydrochloride, lisinopril, metoprolol, pilocarpine, and talcetin, antipsychotic drugs such as chlorpromazine hydrochloride, amitriptyline hydrochloride, nemonapride, haloperidol, moperone hydrochloride, perphenazine, diazepam, lorazepam, chlordiazepoxide, adinazolam, alprazolam, methylphenidate, milnacipran, peroxetin, risperidone, and sodium valproate, anti-emetics such as metoclopramide, ramosetron hydrochloride, granisetron hydrochloride, ondansetron hydrochloride, and azasetron hydrochloride, antihistamines such as chlorpheniramine maleate and diphenhydramine hydrochloride, vitamins suh as thiamine nitrate, tocopherol acetate, cycothiamine, pyridoxal phosphate, cobamamide, ascorbic acid, and nicotinamide, anti-gout drugs such as allopurinol, colchicine, and probenecid, anti-Parkinson's disease drugs such as levodopa and selegiline, sedatives and hypnotics such as amobarbital, bromovaleryl urea, midazolam, and chloral hydrate, antineoplastics such as fluorouracil, carmofur, aclarubicin hydrochloride, cyclophosphamide, and thiotepa, anti-allergy drugs such as pseudoephedrine and terfenadine, decongestants such as phenylpropanolamine and ephedorine, diabetes mellitus drugs such as acetohexamide, insulin, tolbutamide, desmopressin, and glipizide, diuretics such as hydrochlorothiazide, polythiazide, and triamterene, bronchodilators such as aminophylline, formoterol fumarate, and theophylline, antitussives such as codeine phosphate, noscapine, dimemorfan phosphate, and dextromethorphan, anti-arrhythmics, such as quinidine nitrate, digitoxin, propafenone hydrochloride, and procainamide, topical anesthetics such as ethyl aminobenzoate, lidocaine, and dibucaine hydrochloride, anti-convulsants such as phenytoin, ethosuximide, and primidone, synthetic glucocorticoids such as hydrocortisone, prednisolone, triamcinolone, and betamethasone, antiulceratives such as famotidine, ranitidine hydrochloride, cimetidine, sucralfate, sulpiride, teprenone, plaunotol, 5-aminosalicylic acid, sulfasalazine, omeprazole, and lansoprazol, central nervous system drugs, such as indeloxazine, idebenone, tiapride hydrochloride, bifemelane hydrochloride, and calcium pantothenate, antihyperlipoproteinemia such as pravastatin sodium, simvastatin, lovastatin, and atorvastatin, antibiotics such as ampicillin hydrochloride, phthalylsulfacetamide, cefotetan, and josamycin, BPH therapeutic agents such as tamsulosin hydrochloride, doxazosin mesylate, and terazosin hydrochloride, drugs affecting uterine motility such as pranlukast, zafirlukast, albuterol, ambroxol, budesonide, and reproterol, peripheral circulation improvers of prostaglandin I derivatives such as beraprost sodium, anticoagulants, hypotensives, agents for treatment of cardiac insufficiency, agents used to treat the various complications of diabetes, peptic ulcer therapeutic agents, skin ulcer therapeutic agents, agents used to treat hyperlipemia, tocolytics, etc., can be mentioned as such a drug. The drug can be used in its free form or as a pharmaceutically acceptable salt. Moreover, one or a combination of two or more drugs can be used in the present invention.

There are no particular restrictions to the ratio in which the drug used in the present invention is added as long as it is the amount that is normally used pharmacologically for treatment or for prophylaxis, but it is preferably 85 wt% or less, particularly 80 wt% or less, of the entire preparation.

There are no special restrictions to the polyethylene oxide used in the present inventions as long as it can control release of the drug from the preparation. Examples of this polyethylene oxide (also referred to below as PEO) include POLYOX® WSR-303 (viscosity-average molecular weight: 7,000,000, viscosity: 7,500 - 10,000 cP (centipoise: 1% aqueous solution at 25°C)), POLYOX® WSR Coagulant (viscosity-average molecular weight: 5,000,000, viscosity: 5,500 - 7,500 cP (1% aqueous solution at 25°C)), POLYOX® WSR-301 (viscosity-average molecular weight: 4,000,000, viscosity: 1,650-5,500 cP (1% aqueous solution at 25°C)), and POLYOX® WSR N-60K (viscosity-average molecular weight: 2,000,000, viscosity: 2,000 - 4,000 cP (2% aqueous solution at 25°C)) (all made by Union Carbide), ALKOX® E-75 (viscosity-average molecular weight: 2,000,000 to 2,500,000, viscosity: 40 - 70 cP (0.5% aqueous solution at 25°C)), ALKOX® E-100 (viscosity-average molecular weight: 2,500,000 to 3,000,000, viscosity: 90 - 110 cP (0.5% aqueous solution at 25°C)), ALKOX® E-130 (viscosity-average molecular weight: 3,000,000 to 3,500,000, viscosity: 130 - 140 cP (0.5% aqueous solution at 25°C)), ALKOX® E-160 (viscosity-average molecular weight: 3,600,000 to 4,000,000, viscosity: 150 - 160 cP (0.5% aqueous solution at 25°C)), and ALKOX® E-240 (viscosity-average molecular weight: 4,000,000 to 5,000,000, viscosity: 200 - 240 cP (0.5% aqueous solution at 25°C)) (all made by Meisei Kagaku (Chemical)), PEO-8 (viscosity-average molecular weight: 1,700,000 to 2,200,000, viscosity: 20 - 70 cP (0.5% aqueous solution at 25°C)), PEO-15 (viscosity-average molecular weight: 3,300,000 to 3,800,000, viscosity: 130 - 250 cP (0.5% aqueous solution at 25°C)), and PEO-18 (viscosity-average molecular weight: 4,300,000 to 4,800,000, viscosity: 250 - 480 cP (0.5% aqueous solution at 25°C)) (all made by Seitetsu Kagaku (Chemical Industry) Co., Ltd.), etc. Moreover, the PEO used in the present invention preferably has a high viscosity at the time of gelling, or has a high viscosity-average molecular weight. This PEO is preferably, for instance, one with a viscosity of 2,000 cP or higher as an aqueous 2 % solution (25°C) or one that has a viscosity-average molecular weight of 2,000,000 or higher. One or a combination of two or more with different molecular weights, grades, etc., can be used as the PEO of the present invention.

There are no special restrictions to the ratio of polyethylene oxide added in the present invention as long as it is the amount with which release of drug from the preparation usually can be controlled. However, 10 to 95 wt% in terms of total preparation, or 15 to 90 wt% in terms of total preparation, is preferably used. The amount of PEO added is preferably 70 mg or more, particularly 100 mg or more, per 1 unit preparation.

The pharmaceutical composition for oral use according to the present invention is a matrix type sustained-release preparation containing a drug, a hydrophilic base, and polyethylene oxide, wherein yellow ferric oxide and/or red ferric oxide are/is added as described in claim 1 or 4 or 8. The mechanism of this preparation is discussed in International Publication pamphlet WO 94/06414. Because the preparation absorbs water when retained in the upper digestive tract and thereby almost completely gels (not less than 70%, preferably not less than 80%) and moves to the lower digestive tract as the surface of the preparation is being eroded with drug being released by further erosion, the drug is continually and thoroughly released and absorbed. As a result, sustained release performance is realized, even in the colon where there is little water.

There are no particular restrictions to the hydrophilic base as long as it can be dissolved before the polyethylene oxide used in the present invention gels. The amount of water needed to dissolve 1 g of this hydrophilic base is preferably 5 ml or less (at 20 ± 5°C), more preferably 4 ml or less (at the same temperature). Examples of said hydrophilic base include water-soluble polymers such as polyethylene glycol (for instance, Macrogol 400, Macrogol 1500, Macrogol 4000, Macrogol 6000, and Macrogol 20000 (all made by Nihon Yushi)), and polyvinyl pyrrolidone (for instance, PVP® K30 (BASF)), sugar alcohols such as D-sorbitol and xylitol, saccharides such as sucrose, maltose, lactulose, D-fructose, dextran (for instance, Dextran 40), and glucose, surfactants such as polyoxyethylene hydrogenated castor oil (for instance, Cremophor® RH40 (BASF) HCO-40, HCO-60 (Nikko Chemicals), polyoxyethylene polyoxypropylene glycol (for instance, Pluronic® F68 (Asahi Denka), etc.) or polyoxyethylene sorbitan higher fatty acid esters (such as Tween 80 (Kanto Kagaku(Chemical)), etc.), salts such as sodium chloride and magnesium chloride, organic acids such as citric acid and tartaric acid, amino acids such as glycine, β-alanine, lysine hydrochloride, and amino saccharides such as meglumine, etc. Polyethylene glycol, sucrose and polyvinyl pyrrolidone are preferred and polyethylene glycol (particularly Macrogol 6000) is further preferred. Moreover, one or a combination of two or more hydrophilic bases can be used in the present invention.

The ratio of the hydrophilic base used in the present invention, is preferably 5 to 80 wt%, particularly 5 to 60 wt%, per total preparation.

The yellow ferric oxide and/or red ferric oxide used in the present invention can be used alone or in combination.

There are no special restrictions to the ratio at which yellow ferric oxide and/or red ferric oxide of the present invention is added as long as it is an amount that can stabilize a matrix type sustained-release preparation and reduce changes in drug release profile. This compounding ratio differs depending on the type of substance and the method of addition, but it is preferably 1 to 20 wt%, particularly 3 to 15 wt%, in the case of physical mixing in the matrix per preparation weight. For instance, as for red ferric oxide, it is preferably 5 to 20 wt%, particularly 10 to 15 wt%, per preparation weight. As for yellow ferric oxide, it is preferably 1 to 20 wt%, particularly 3 to 10 wt%, per preparation weight. When used in film coating, it is preferably 0.3 to 2 %, particularly 0.5 to 1.5 %, per tablet weight. The concentration of yellow ferric oxide or red ferric oxide contained in the film is preferably 5 to 50 %, more preferably 10 to 20 %.

The term "physical mixing in the matrix" used here means that the drug and yellow ferric oxide and/or red ferric oxide are uniformly dispersed in PEO as the main base of the sustained-release preparation. Moreover, the term "film coating" means that, for instance, the ferric oxide is dissolved or suspended in a water-soluble polymer solution of hydroxypropylmethyl cellulose, etc., and this is coated as a thin film to a tablet that has been separately prepared. Yellow ferric oxide and/or red ferric oxide in the present invention can exist at any place in the preparation. For instance, it can be contained in the film by film coating, in granules by granulation or in the matrix (for instance, around the polyethylene oxide).

There are no special restrictions to the reduction by the present invention of changes in drug release from a pharmaceutical composition for oral use containing polyethylene oxide, as long as the yellow ferric oxide and/or red ferric oxide is added. For instance, the means of film coating, granulation, mixing, etc. are mentioned, and can be performed singly or in a combination of two or more.

Other additives that are pharmaceutically acceptable can be added as needed to the pharmaceutical composition of the present invention. For instance, one or a combination of two or more of fillers such as lactose, mannitol, potato starch, wheat starch, rice starch, corn starch, crystalline cellulose, etc., binders such as hydroxypropylmethyl cellulose, hydroxypropyl cellulose, methyl cellulose, gum Arabic, etc., swelling agents such as carboxymethyl cellulose, carboxymethyl cellulose calcium, cross-linking carboxymethylcellulose sodium, etc., lubricants such as stearic acid, calcium stearate, magnesium stearate, talc, magnesium metasilicoaluminate, calcium hydrogen phosphate, anhydrous calcium hydrogen phosphate, etc., fluidizers such as silicon dioxide hydrate, light silic anhydride, dry aluminum hydroxide gel, etc., coloring agents such as yellow ferric oxide, ferric oxide, etc., surfactants such as sodium laurylsulfate, sucrose fatty acid esters, etc., coating agents such as zein, hydroxypropyl methyl cellulose, hydroxypropyl cellulose, etc., flavorings such as 1-menthol, mentha oil, fennel oil, etc., preservatives such as sodium sorbate, potassium sorbate, methyl parabenzoate, ethyl parabenzoate, etc., buffers such as citric acid, succinic acid, fumaric acid, tartaric acid, ascorbic acid, salts thereof, glutamic acid, glutamine, glycine, aspartic acid, alanine, arginine and its salts, magnesium oxide, zinc oxide, magnesium hydroxide, phosphoric acid, boric acid, its salts, etc., can be added as needed.

There are no special restrictions to the method of the present invention as long as it is a method that is usually applicable for hydrogel preparations for instance, the tableting method whereby yellow ferric oxide and/or red ferric oxide and various additives as needed, are mixed with the drug hydrophilic base, and PEO, and the mixture is compression molded; capsule compress on filling; extrusion molding, or injection molding, wherein the mixture is melted and solidified. In addition, coating, such as conventional sugar coating and film coating after molding, can be performed as needed. It is also possible to fill the product into capsules after molding.

## BEST MODE FOR CARRYING OUT THE INVENTION

The present invention will now be explained in further detail while referring to a Comparative Example, Examples and Test Examples, but the present invention is not construed as being limited thereto.

[Comparative Example]

| Polyethylene oxide (Polyox® WSR303) | 150 (parts by weight) |
|---|---|
| Macrogol 6000 | 30 |

The polyethylene oxide and Macrogol 6000 were mixed with a mortar and pestle and made into tablets with an oil press at a tableting pressure of 1 ton/punch to obtain uncoated tablets with a diameter of 8 mm and tablet weight of 180 mg.

[Example 1]

| Polyethylene oxide (Polyox® WSR303) | 150 (parts by weight) |
|---|---|
| Macrogol 6000 | 30 |

The polyethylene oxide and Macrogol 6000 were mixed with a mortar and pestle and made into tablets with an oil press at a tableting pressure of 1 ton/punch to obtain tablets with a diameter of 8 mm and tablet weight of 180 mg.
8.0 g of hydroxypropylmethyl cellulose 2910 (TC-5R) and 1.5 g Macrogol 6000 were dissolved in 88.5 g purified water and then 2.0 g yellow ferric oxide were dispersed therein to obtain a coating liquid. The uncoated tablets that had been obtained were coated with 3% of this solution per tablet weight using a film coating device (HCT-mini, Freund Sangyo) to obtain the tablets of the present invention.

[Example 2]

The uncoated tablets obtained in Example 1 was film-coated with the following coating solution:

8.0g of hydroxypropylmethyl cellulose 2910 (TC-5R) and 1.5 g Macrogol 6000 were dissolved in 88.5 g purified water and then 2.0 g red ferric oxide were dispersed therein to obtain a coating liquid. The uncoated tablets that had been obtained in Example 1 were coated with 3% of this solution per tablet weight using a film coating device (HCT-mini, Freund Sangyo) to obtain the tablets of the present invention.

[Example 3]

The uncoated tablets obtained in Example 1 was film-coated with the following coating solution:

8.0 g of hydroxypropylmethyl cellulose 2910 (TC-5R) and 1.5 g Macrogol 6000 were dissolved in 88.5 g purified water and then 2.0 g yellow ferric oxide and 0.5 g titanium oxide were dispersed therein to obtain a coating liquid. The uncoated tablets that had been obtained were coated with 3% of this solution per tablet weight using a film coating device (HCT-mini, Freund Sangyo) to obtain the tablets of the present invention.

[Example 4]

The uncoated tablets obtained in Example 1 was film-coated with the following coating solution:

8.0 g of hydroxypropylmethyl cellulose 2910 (TC-5R) and 1.5 g Macrogol 6000 were dissolved in 88.5 g purified water and then 2.0 g red ferric oxide and 0.5 g titanium oxide were dispersed therein to obtain a coating liquid. The uncoated tablets that had been obtained were coated with 3 % of this solution per tablet weight using a film coating device (HCT-mini, Freund Sangyo) to obtain the tablets of the present invention.

[Example 5]

| Polyethylene oxide (Polyox® WSR303) | 150 (parts by weight) |
| Macrogol 6000 | 30 |
| Red ferric oxide | 20 |

The polyethylene oxide, Macrogol 6000 and red ferric oxide were mixed with a mortar and pestle and then made into tablets using an oil press at a tableting pressure of 1 ton/punch to obtain the tablets of the invention with a diameter of 8 mm and a tablet weight of 200 mg.

[Example 6]

| Polyethylene oxide (Polyox® WSR303) | 150 (parts by weight) |
| Macrogol 6000 | 30 |
| Yellow ferric oxide | 20 |

The polyethylene oxide, Macrogol 6000, and yellow ferric oxide were mixed with a mortar and pestle and then made into tablets using an oil press at a tableting pressure of 1 ton/punch to obtain tablets of the present invention with a diameter of 8 mm and tablet weight of 200 mg.

[Example 7]

| Polyethylene oxide (Polyox® WSR303) | 150 (parts by weight) |
| Macrogol 6000 | 30 |
| Yellow ferric oxide | 9.5 |

The polyethylene oxide, Macrogol 6000, and yellow ferric oxide were mixed with a mortar and pestle and then made into tablets using an oil press at a tableting pressure of 1 ton/punch to obtain tablets of the present invention with a diameter of 8 mm and tablet weight of 189.5 mg.

[Test Example] (Stability during storage under exposure to light)

The tablets obtained in the Comparative Example and Examples 1 through 7 were introduced to a plastic dish and exposed to light. Light exposure was performed by exposure to light for 8 weeks using Option 1 according to ICH guidelines (D65, which is the international standard for outdoor light in accordance with ISO10977) so that the total illumination intensity would be 1,200,000 Lux - hr. The following experiment was performed using tablets that had been exposed to light and tablets that had been stored for the same amount of time but under shaded condition.

Matrix erosion experiment

The experiment was performed with a paddle speed at 200 rpm in accordance with Japan Pharmacopoeia Dissolution Experimental Methods, Method No. 2 (Paddle Method) using 500 ml purified water as the experimental solution. Six hours after starting the experiment, the tablets were removed from the flask and dried for 4 days in a dryer at 40°C to evaporate the water content of the matrix tablet. The matrix erosion percentage was calculated from the difference between initial weight of the tablet and dry weight using equation (I) (Table 1).

Matrix erosion percentage =
(initial weight - dry weight) / initial weight x 100     (equation I)

Table 1. Matrix erosion experiment results

| Preparation | Erosion Percentage (%) | |
|---|---|---|
| | Shaded from light | Exposed to 1,200,000 Lux · h |
| Comparative Example 1 | 39.7 ± 0.4 | 57.4 ± 2.1 |
| Example 1 | 42.0 ± 0.3 | 42.6 ± 0.0 |
| Example 2 | 42.3 ± 1.0 | 41.5 ± 0.1 |
| Example 3 | 42.4 ± 0.5 | 42.8 ± 0.4 |
| Example 4 | 42.4 ± 0.5 | 42.8 ± 0.1 |
| Example 5 | 42.8 ± 0.5 | 47.7 ± 0.3 |
| Example 6 | 41.8 ± 0.2 | 41.6 ± 0.2 |
| Example 7 | 41.5 ± 0.4 | 42.8 ± 0.2 |

(n=3, mean ± SD)

Results and Discussion

As shown in Table 1, it was expected that the drug release rate of the Comparative Example in which the preparation did not contain yellow ferric oxide and/or red ferric oxide would be accelerated with exposure to light because the matrix erosion percentage increased. The matrix erosion percentage was the same levelas with the storage with shading from light using film coating containing yellow ferric oxide or red ferric oxide. Moreover, acceleration of the matrix erosion percentage was prevented, and particularly strong results were obtained with the addition of yellow ferric oxide, when physical mixing with yellow ferric oxide or red ferric oxide was performed. Consequently, these results indicate that the pharmaceutical composition for oral use of the present invention to which yellow ferric oxide or red ferric oxide has been added is a stable preparation that shows no changes in drug release profile during storage even under exposure to light.

INDUSTRIAL APPLICABILITY

The pharmaceutical composition for oral use and the method of producing said composition of the present invention is to provide a stable preparation and a method of producing a stable preparation in which there is no changes in drug release profile in a matrix type sustained-release preparations containing polyethylene oxide, during storage even under exposure to light.

The pharmaceutical composition for oral use is to provide a stable pharmaceutical composition for oral use by adding by means of coating or by physical mixture yellow ferric oxide and/or red ferric oxide, in which there is no changes in drug release profile. The method of stabilizing by means of mixture is to simplify the step of production processes, because the above mentioned substance can be added during granulation with polyethylene oxide and additives.

In addition, the method of the present invention makes it possible to provide a method of preventing changes in drug release profile in a matrix type sustained-release preparation containing a drug, a hydrophilic base, and polyethylene oxide.

Therefore, the quality assurance period of the product can be expected to be prolonged and the product value can be increased as a result of stabilization of sustained-release preparations.

Claims

1.    A stable pharmaceutical composition for oral use which is a matrix type sustained-release preparation and comprises:

(a) a drug,
(b) 5 to 80 wt.% of hydrophilic base having a solubility such that the amount of water needed to dissolve 1g of said hydrophilic base is 5ml or less at 20 ± 5°C,
(c) 10 to 95 wt.% of polyethylene oxide (i) having a viscosity of 2,000 cP or more as an aqueous 2% solution at 25°C or (ii) having a viscosity average molecular weight of 2, 000, 000 or more, said drug and hydrophilic base being dispersed in a matrix of said polyethyelene oxide, and
(d) yellow ferric oxide and/or red ferric oxide, wherein (i) the amount of said yellow ferric oxide and/or red ferric oxide is effective to stabilise the drug release rate of the preparation, and (ii) yellow ferric oxide is present in an amount of 1 to 20% of the weight of the preparation or red ferric oxide is present in an amount of 5 to 20% of the weight of the preparation.

2. A composition according to claim 1 wherein yellow ferric oxide in an amount of 3 to 10% or red ferric oxide in an amount of 10 to 15%, based on the weight of the preparation, is contained by physical mixing in the matrix.

3. A composition according to claim 1 or 2 wherein the content of the polyethylene oxide is from 15 to 90 wt.% of the total preparation.

4. A stable pharmaceutical tablet for oral use which is a matrix type sustained-release preparation and comprises:

   (a) a drug,
   (b) 5 to 80 wt.% of hydrophilic base having a solubility such that the amount of water needed to dissolve 1g of said hydrophilic base is 5ml or less at 20 $\pm$ 5°C, (c) 10 to 95 wt.% of polyethylene oxide (i) having a viscosity of 2,000 cP or higher as an aqueous 2% solution at 25°C or (ii) having a viscosity average molecular weight of 2,000,000 or higher, said drug and hydrophilic base being dispersed in a matrix of said polyethylene oxide, and
   (d) a tablet film coating containing yellow ferric oxide and/or red ferric oxide in an amount which is 0.3 to 2% of the tablet weight and is effective to stabilise the drug release rate of the tablet.

5. A tablet according to claim 4 wherein the amount of yellow ferric oxide and/or red ferric oxide in the film coating is 0.5 to 1.5% of the tablet weight.

6. A tablet according to claim 4 or 5 wherein the concentration of yellow ferric oxide and/or red ferric oxide in the film coating is 5 to 50%.

7. A tablet according to any of claims 4 to 6 wherein the content of the polyethylene oxide is from 15 to 90 wt.% of the total preparation.

8. A method of producing a stable pharmaceutical composition for oral use which is a matrix type sustained-release preparation, which method comprises combining:

   (a) a drug,
   (b) 5 to 80 wt.% of hydrophilic base having a solubility such that the amount of water needed to dissolve 1g of said hydrophilic base is 5ml or less at 20 $\pm$ 5°C,
   (c) 10 to 95 wt.% of polyethylene oxide (i) having a viscosity of 2,000cP or more as an aqueous 2% solution at 25°C or (ii) having a viscosity average molecular weight of 2, 000, 000 or more, said drug and hydrophilic base being dispersed in a matrix of said polyethylene oxide, and
   (d) yellow ferric oxide and/or red ferric oxide, wherein
   (i) the amount of said yellow ferric oxide and/or red ferric oxide is effective to stabilise the drug release rate of the preparation, and (ii) yellow ferric oxide is present in an amount of 1 to 20% of the weight of the preparation or red ferric oxide is present in an amount of 5 to 20% of the weight of the preparation, or tabletting said ingredients (a) to (c) and providing each tablet with a film coating containing yellow ferric oxide and/or red ferric oxide in an amount which is 0.3 to 2% of the tablet weight and is effective to stabilise the drug release rate of the tablet.

9. A method according to claim 8 wherein yellow ferric oxide and/or red ferric oxide is physically mixed in the matrix in an amount of from 1 to 20% of the weight of the preparation.

10. A method according to claim 8 or 9 wherein from 3 to 10% of yellow ferric oxide or from 10 to 15% of red ferric oxide, based on the weight of the preparation, is physically mixed in the matrix.

11. A method according to any of claims 8 to 10 wherein the amount of the polyethylene oxide used is from 15 to 90 wt.% of the total preparation.

12. The use of yellow ferric oxide and/or red ferric oxide to stabilise an oral matrix type sustained release preparation containing drug, hydrophilic base and polyethylene oxide against storage-induced acceleration of drug release.

**13.** Use according to claim 12 wherein said yellow ferric oxide and/or red ferric oxide is present in said preparation in a film coating or by way of granulation or physical mixing in the matrix.

**Patentansprüche**

**1.** Stabile pharmazeutische Zusammensetzung zur oralen Verwendung, bei der es sich um eine Zubereitung mit verzögerter Freigabe vom Matrixtyp handelt, und die umfaßt:

(a) ein Arzneimittel,
(b) 5 bis 80 Gew.% einer hydrophilen Base mit einer solchen Löslichkeit, daß die Menge des benötigten Wassers, um 1 g der hydrophilen Base zu lösen, bei 20 ± 5°C 5 ml oder weniger beträgt,
(c) 10 bis 95 Gew.% Polyethylenoxid (i) mit einer Viskosität von 2.000 cP oder mehr als wäßrige 2 % Lösung bei 25°C oder (ii) mit einer viskositätsgemittelten Molekülmasse von 2.000.000 oder mehr, wobei das Arzneimittel und die hydrophile Base in einer Matrix des Polyethylenoxids dispergiert sind, und
(d) gelbes Eisen(III)oxid und/oder rotes Eisen(III)oxid, worin (i) die Menge des gelben Eisen(III)oxids und/oder roten Eisen(III)oxids zur Stabilisierung der Arzneimittelfreigabegeschwindigkeit der Zubereitung wirksam ist und (ii) gelbes Eisen(III)oxid in einer Menge von 1 bis 20 Gew.% der Zubereitung vorhanden ist oder rotes Eisen(III)oxid in einer Menge von 5 bis 20 Gew.% der Zubereitung vorhanden ist.

**2.** Zusammensetzung gemäß Anspruch 1, worin gelbes Eisen(III)oxid in einer Menge von 3 bis 10 % oder rotes Eisen (III)oxid in einer Menge von 10 bis 15 % auf Basis des Gewichts der Zubereitung durch physikalisches Mischen in der Matrix enthalten ist.

**3.** Zusammensetzung gemäß Anspruch 1 oder 2, worin der Gehalt des Polyethylenoxids 15 bis 90 Gew.% der Gesamtzubereitung beträgt.

**4.** Stabile pharmazeutische Tablette zur oralen Verwendung, bei der es sich um eine Zubereitung mit verzögerter Freigabe vom Matrixtyp handelt, und die umfaßt:

(a) ein Arzneimittel,
(b) 5 bis 80 Gew.% einer hydrophilen Base mit einer solchen Löslichkeit, daß die Menge des benötigten Wassers, um 1 g der hydrophilen Base zu lösen, bei 20 ± 5°C 5 ml oder weniger beträgt,
(c) 10 bis 95 Gew.% Polyethylenoxid (i) mit einer Viskosität von 2.000 cP oder mehr als wäßrige 2 % Lösung bei 25°C oder (ii) mit einer viskositätsgemittelten Molekülmasse von 2.000.000 oder mehr, wobei das Arzneimittel und die hydrophile Base in einer Matrix des Polyethylenoxids dispergiert sind, und
(d) eine Tablettenfilmbeschichtung enthaltend gelbes Eisen (III) oxid und/oder rotes Eisen(III)oxid in einer Menge, die 0,3 bis 2 % des Tablettengewichts ausmacht und die zum Stabilisieren der Arzneimittelfreigabegeschwindigkeit der Tablette wirksam ist.

**5.** Tablette gemäß Anspruch 4, worin die Menge an gelbem Eisen(III)oxid und/oder rotem Eisen(III)oxid in der Filmbeschichtung 0,5 bis 1,5 % des Tablettengewichts beträgt.

**6.** Tablette gemäß Anspruch 4 oder 5, worin die Konzentration an gelbem Eisen (III) oxid und/oder rotem Eisen(III) oxid in der Filmbeschichtung 5 bis 50 % beträgt.

**7.** Tablette gemäß einem der Ansprüche 4 bis 6, worin der Gehalt des Polyethylenoxids 15 bis 90 Gew. % der Gesamtzubereitung beträgt.

**8.** Verfahren zur Herstellung einer stabilen pharmazeutischen Zusammensetzung zur oralen Verwendung, bei der es sich um eine Zubereitung mit verzögerter Freigabe vom Matrixtyp handelt, wobei das Verfahren das Kombinieren umfaßt von:

(a) einem Arzneimittel,
(b) 5 bis 80 Gew.% einer hydrophilen Base mit einer solchen Löslichkeit, daß die Menge des benötigten Wassers, um 1 g der hydrophilen Base zu lösen, bei 20 ± 5°C 5 ml oder weniger beträgt,
(c) 10 bis 95 Gew.% Polyethylenoxid (i) mit einer Viskosität von 2.000 cP oder mehr als wäßrige 2 % Lösung bei 25°C oder (ii) mit einer viskositätsgemittelten Molekülmasse von 2.000.000 oder mehr, wobei das Arzneimittel und die hydrophile Base in einer Matrix des Polyethylenoxids dispergiert sind, und

(d) gelbem Eisen(III)oxid und/oder rotem Eisen(III)oxid, worin (i) die Menge des gelben Eisen (III) oxids und/oder roten Eisen(III)oxids zur Stabilisierung der Arzneimittelfreigabegeschwindigkeit der Zubereitung wirksam ist und (ii) gelbes Eisen(III)oxid in einer Menge von 1 bis 20 Gew.% der Zubereitung vorhanden ist oder rotes Eisen(III)oxid in einer Menge von 5 bis 20 Gew.% der Zubereitung vorhanden ist, oder Tablettieren des Inhaltsstoffe (a) bis (c) und Bereitstellen jeder Tablette mit einer Filmbeschichtung enthaltend gelbes Eisen(III)oxid und/oder rotes Eisen(III)oxid in einer Menge, die 0,3 bis 2 % des Tablettengewichts ausmacht, und die zum Stabilisieren der Arzneimittelfreisetzungsgeschwindigkeit der Tablette wirksam ist.

9. Verfahren gemäß Anspruch 8, worin gelbes Eisen(III)oxid und/oder rotes Eisen(III)oxid in einer Menge von 1 bis 20 % des Gewichts der Zubereitung in die Matrix physikalisch vermischt wird.

10. Verfahren gemäß Anspruch 8 oder 9, worin 3 bis 10 % des gelben Eisen(III)oxids und/oder 10 bis 15 % roten Eisen (III)oxids auf Basis des Gewichts der Zubereitung in der Matrix physikalisch gemischt wird.

11. Verfahren gemäß einem der Ansprüche 8 bis 10, worin die Menge des verwendeten Polyethylenoxids 15 bis 90 Gew.% der Gesamtzubereitung beträgt.

12. Verwendung von gelbem Eisen(III)oxid und/oder rotem Eisen(III)oxid zur Stabilisierung einer oralen Zubereitung mit verzögerter Freigabe vom Matrixtyp enthaltend Arzneimittel, hydrophile Base und Polyethylenoxid, gegen lagerungsinduzierte Beschleunigung der Arzneimittelfreigabe.

13. Verwendung gemäß Anspruch 12, worin das gelbe Eisen(III)oxid und/oder rote Eisen(III)oxid in der Zubereitung in einer Filmbeschichtung oder durch Granulieren oder physikalisches Mischen in der Matrix vorhanden ist.

## Revendications

1. Composition pharmaceutique stable pour une utilisation orale, qui est une préparation à libération soutenue du type matrice et qui comprerid :

(a) un médicament,
(b) 5 à 80 % en masse de base hydrophile ayant une solubilité telle que la quantité d'eau nécessaire pour dissoudre 1 g de ladite base hydrophile soit égale ou inférieure à 5 ml à 20 $\pm$ 5°C,
(c) 10 à 95 % en masse d'oxyde de polyéthylène (i) ayant une viscosité, en solution aqueuse à 2 % à 25°C, égale ou supérieure à 2 000 cP ou (ii) ayant une masse moléculaire moyenne en viscosité égale ou supérieure à 2 000 000, ledit médicament et ladite base hydrophile étant dispersés dans une matrice dudit oxyde de polyéthylène, et
(d) de l'oxyde ferrique jaune et/ou de l'oxyde ferrique rouge, où (i) la quantité dudit oxyde ferrique jaune et/ou dudit oxyde ferrique rouge est efficace pour stabiliser la vitesse de libération du médicament de la préparation et (ii) l'oxyde ferrique jaune est présent en une quantité de 1 à 20 % en masse par rapport à la préparation ou l'oxyde ferrique rouge est présent en une quantité de 5 à 20 % en masse par rapport à la préparation.

2. Composition selon la revendication 1, dans laquelle l'oxyde ferrique jaune, en une quantité de 3 à 10 %, ou l'oxyde ferrique rouge, en une quantité de 10 à 15 %, en masse par rapport à la préparation, est contenu dans la matrice par mélange physique.

3. Composition selon la revendication 1 ou 2, dans laquelle le taux de l'oxyde de polyéthylène est entre 15 et 90% en masse par rapport à la préparation totale.

4. Composition pharmaceutique stable pour une utilisation orale, qui est une préparation à libération soutenue du type matrice et qui comprend :

(a) un médicament,
(b) 5 à 80 % en masse de base hydrophile ayant une solubilité telle que la quantité d'eau nécessaire pour dissoudre 1 g de ladite base hydrophile soit égale ou inférieure à 5 ml à 20 $\pm$ 5°C,
(c) 10 à 95 % en masse d'oxyde de polyéthylène (i) ayant une viscosité, en solution aqueuse à 2 % à 25°C, égale ou supérieure à 2 000 cP ou (ii) ayant une masse moléculaire moyenne en viscosité égale ou supérieure à 2 000 000, ledit médicament et ladite base hydrophile étant dispersés dans une matrice dudit oxyde de polyéthylène, et

(d) un enrobage par film pour comprimé contenant de l'oxyde ferrique jaune et/ou de l'oxyde ferrique rouge, en une quantité de 0,3 à 2 % en masse par rapport au comprimé et efficace pour stabiliser la vitesse de libération du médicament du comprimé.

5. Comprimé selon la revendication 4, dans lequel la quantité d'oxyde ferrique jaune et/ou d'oxyde ferrique rouge dans l'enrobage par film est de 0,5 à 1,5 % par rapport à la masse du comprimé.

6. Comprimé selon la revendication 4 ou 5, dans lequel la concentration de l'oxyde ferrique jaune et/ou de l'oxyde ferrique rouge dans le film d'enrobage est de 5 à 50 %.

7. Comprimé selon l'une quelconque des revendications 4 à 6, dans lequel le taux de l'oxyde de polyéthylène est de 15 à 90% en masse par rapport à la préparation totale.

8. Méthode de production d'une composition pharmaceutique stable pour une utilisation orale, qui est une préparation à libération soutenue du type matrice, cette méthode comprenant la combinaison :

(a) d'un médicament,
(b) de 5 à 80 % en masse de base hydrophile ayant une solubilité telle que la quantité d'eau nécessaire pour dissoudre 1 g de ladite base hydrophile soit égale ou inférieure à 5 ml à 20 $\pm$ 5°C,
(c) de 10 à 95 % en masse d'oxyde de polyéthylène (i) ayant une viscosité, en solution aqueuse à 2 % à 25°C, égale ou supérieure à 2 000 cP ou (ii) ayant une masse moléculaire moyenne en viscosité égale ou supérieure à 2 000 000, ledit médicament et ladite base hydrophile étant dispersés dans une matrice dudit oxyde de polyéthylène, et
(d) d'oxyde ferrique jaune et/ou d'oxyde ferrique rouge, où (i) la quantité dudit oxyde ferrique jaune et/ou dudit oxyde ferrique rouge est efficace pour stabiliser la vitesse de libération du médicament de la préparation et (ii) l'oxyde ferrique jaune est présent en une quantité de 1 à 20 % en masse par rapport à la préparation ou l'oxyde ferrique rouge est présent en une quantité de 5 à 20 % en masse par rapport à la préparation, ou la compression desdits ingrédients (a) à (c) et l'enrobage de chaque comprimé par un film contenant de l'oxyde ferrique jaune et/ou de l'oxyde ferrique rouge, en une quantité de 0,3 à 2 % par rapport à la masse du comprimé et efficace pour stabiliser la vitesse de libération du médicament du comprimé.

9. Méthode selon la revendication 8, dans laquelle de l'oxyde ferrique jaune et/ou de l'oxyde ferrique rouge est mélangé physiquement dans la matrice en une quantité de 1 à 20 % par rapport à la masse du comprimé.

10. Méthode selon la revendication 8 ou 9, dans laquelle de 3 à 10 % d'oxyde ferrique jaune ou de 10 à 15 % d'oxyde ferrique rouge, par rapport à la masse du comprimé, est mélangé physiquement dans la matrice.

11. Méthode selon l'une quelconque des revendications 8 à 10, dans lequel la quantité de l'oxyde de polyéthylène utilisée est de 15 à 90% en masse par rapport à la préparation totale.

12. Utilisation d'oxyde ferrique jaune et/ou d'oxyde ferrique rouge pour stabiliser, contre l'accélération de la libération du médicament induite par le stockage, une préparation orale à libération soutenue du type matrice contenant un médicament, une base hydrophile et de l'oxyde de polyéthylène.

13. Utilisation selon la revendication 12, dans laquelle ledit oxyde ferrique jaune et/ou ledit oxyde ferrique rouge est présent dans ladite revendication dans un film d'enrobage ou, grâce à une granulation ou un mélange physique, dans la matrice.